Europäisches Patentamt

European Patent Office

Office européen des brevets

(11) Veröffentlichungsnummer: **0 289 906 B1**

(12) **EUROPÄISCHE PATENTSCHRIFT**

(45) Veröffentlichungstag der Patentschrift: **30.12.92**

(21) Anmeldenummer: **88106612.0**

(22) Anmeldetag: **25.04.88**

(51) Int. Cl.⁵: **A61N 1/04**, A61B 5/04

(54) **Unterdruckdüse für Saugelektroden.**

�úthirtieth Priorität: **08.05.87 DE 8706666 U**

(43) Veröffentlichungstag der Anmeldung:
**09.11.88 Patentblatt 88/45**

(45) Bekanntmachung des Hinweises auf die
Patenterteilung:
**30.12.92 Patentblatt 92/53**

(84) Benannte Vertragsstaaten:
**AT BE CH DE GB LI NL**

(56) Entgegenhaltungen:
**EP-A- 0 000 759**
**DE-B- 1 939 523**
**DE-C- 3 300 765**

**ELEKTROMEDICA, Band 39, Nr. 4, April 1971,
Seite III, Erlangen, DE; G. HENKE:
"Vereinfachte und sichere Elektrodenanlage
in der Reizstrombehandlung durch Appliserv"**

(73) Patentinhaber: **SIEMENS AKTIENGESELL-
SCHAFT**
**Wittelsbacherplatz 2**
**W-8000 München 2(DE)**

(72) Erfinder: **Sieber, Gotthold**
**Hedenusstrasse 29**
**W-8520 Erlangen(DE)**
Erfinder: **Zachäus, Edgar**
**Atriumweg 1**
**W-6233 Kelkheim/Taunus(DE)**

**Beschreibung**

Die Erfindung bezieht sich auf eine Unterdruckdüse gemäß Oberbegriff des Anspruchs 1.

Durch die DE-B-19 39 523 ist eine Saugelektrode für Reizstromtherapie vorbekannt, bei der auf einer Elektrodenplatte ein Metallrohr mit den Merkmalen des Oberbegriffs des Anspruchs 1 als Unterdruckdüse aufgelötet ist. Der Ansaugkanal ist dabei durch die Elektrodenplatte hindurch bis ins Innere des Rohrkanals nahe der engen Stelle gebohrt. Das die Unterdruckdüse bildende Metallrohr sowie die fest damit verbundene Elektrodenplatte sitzen in einem Elektrodengehäuse, das applikationsseitig eine umlaufende Dichtlippe aufweist. Wird die Saugelektrode mit der Dichtlippe auf der Hautoberfläche eines behandelnden Patienten aufgesetzt und wird dann anschließend vom einen Ende der Unterdruckdüse durch eine Gaspumpe beispielsweise elektrisch isolierendes Preßgas durch die Unterdruckdüse geblasen, so entsteht an der engen Stelle ein Unterdruck, der sich über den Ansaugkanal zur Applikationsstelle fortpflanzt. Die Saugelektrode saugt sich daraufhin auf der Hautoberfläche an. Elektrodenkontaktflüssigkeit, mit der z.B. zur Herstellung besseren Kontaktes ein zwischen Elektrodenplatte und Hautoberfläche des Patienten an der Applikationsstelle eingesetzter Elektrodenschwamm getränkt ist und die während der Behandlungszeit wegen des Unterdruckes über den Ansaugkanal in den Rohrkanal angesaugt wird, wird über das frei mündende Ende des Rohrkanals in die Atmosphäre versprüht. Die Elektrodenkontaktflüssigkeit kann also nicht in die Gaspumpe gelangen und diese verschmutzen.

Bei der in der bekannten Saugelektrode verwendeten Unterdruckdüse läßt sich der Ansaugkanal nur schwierig reinigen. Außerdem bereitet die Fertigung der Unteroruckdüse nicht unerhebliche Probleme, weil es nämlich schwierig ist, den Ansaugkanal so zu bohren, daß er im Rohrkanal immer exakt die enge Stelle trifft, wo der Unterdruck erzeugt werden soll.

Aus der DE-C-33 00 765 ist eine Saugelektrode mit einem Elektrodengehäuse bekannt, in dem eine elektrisch leitende Elektrode und eine Unterdruckdüse angeordnet sind, wobei die Unterdruckdüse einen Kanal mit einer verengten Stelle zur Erzeugung eines Unterdruckes aufweist. Die bekannte Vorrichtung ist als Strahlpumpe ausgebildet und mit der Elektrodeneinrichtung zu einer baulichen Einheit verbunden, nämlich unmittelbar an einer Saugglocke angeordnet. Dazu ist die Elektrode an dem die Seitenwand der Saugglocke diametral durchstoßenden Strahlrohr aus Metall befestigt und die Verengung des Strahlrohres steht über einen Ansaugkanal mit dem Inneren der Saugglocke in Verbindung. Da der Ansaugkanal nur durch eine Bohrung im Strahlrohrmantel in das Strahlrohr einmündet, ist die Reinigung des Ansaugkanals der bekannten Vorrichtung entsprechend schwierig.

Aufgabe vorliegender Erfindung ist es, eine Unterdruckdüse für eine Saugelektrode aufzubauen, bei der sich der Ansaugkanal fertigungstechnisch leichter als bisher herstellen läßt und auch eine Reinigung eines verstopften Ansaugkanals sehr viel einfacher und rascher als bisher möglich ist.

Die Aufgabe wird erfindungsgemäß durch die Merkmale des Patentanspruchs 1 gelöst.

Gemäß der Erfindung durchstößt der Ansaugkanal das die Unterdruckdüse bildende Rohr. Damit liegt die enge Stelle nicht direkt über der Mündung des Ansaugkanals, sondern in Richtung des Einpumpeinganges für das Einpumpmedium vor der Mündung des Ansaugkanals. Der Ansaugkanal kann also leicht mittels einer feinen Nadel durchstochen werden, wobei der dabei sich lösende Schmutz vor die Mündung der engen Stelle gedrückt wird und von dort vom durchgepumpten Medium in Richtung frei mündenden Auslauf des Rohrkanals mitgerissen wird. Die Unterdruckdüse mit durchgehendem Ansaugkanal läßt sich auch sehr viel einfacher und billiger herstellen. Zuerst wird nämlich der Ansaugkanal gebohrt, wobei der Rohrrohling bereits vorab mit einem engen Kanal vom Durchmesser der engen Stelle versehen sein kann. Anschließend wird dann der Rohrkanal schrittweise von beiden Rohrenden her in der jeweils gewünschten Form gebohrt. Die enge Stelle mündet dann immer direkt am Ansaugkanal. Die Aufweitung des Rohrkanals zum einlaßseitigen Rohrende hin ist konusförmig erweitert und auslaßseitig kann der Rohrkanal stufenförmig erweitert ausgeführt sein.

Weitere Vorteile und Einzelheiten der Erfindung ergeben sich aus der nachfolgenden Beschreibung eines Ausführungsbeispiels anhand der Zeichnung.

Es zeigen:

Figur 1 eine Saugelektrode, die eine erfindungsgemäße Unterdruckdüse umfaßt,
Figur 2 eine auswechselbare Haube für die Saugelektrode der Figur 1,
Figur 3 die Art der Befestigung der elektrisch leitenden Elektrode der Saugelektrode an der Unterdruckdüse,
Figur 4 ein Ausführungsbeispiel der Unterdruckdüse im Längsschnitt,
Figur 5 eine Draufsicht auf die Unterdruckdüse der Figur 4, teilweise im Schnitt,
Figuren 6 bis 14 Verfahrensschritte zur Herstellung einer erfindungsgemäßen Unterdruckdüse.

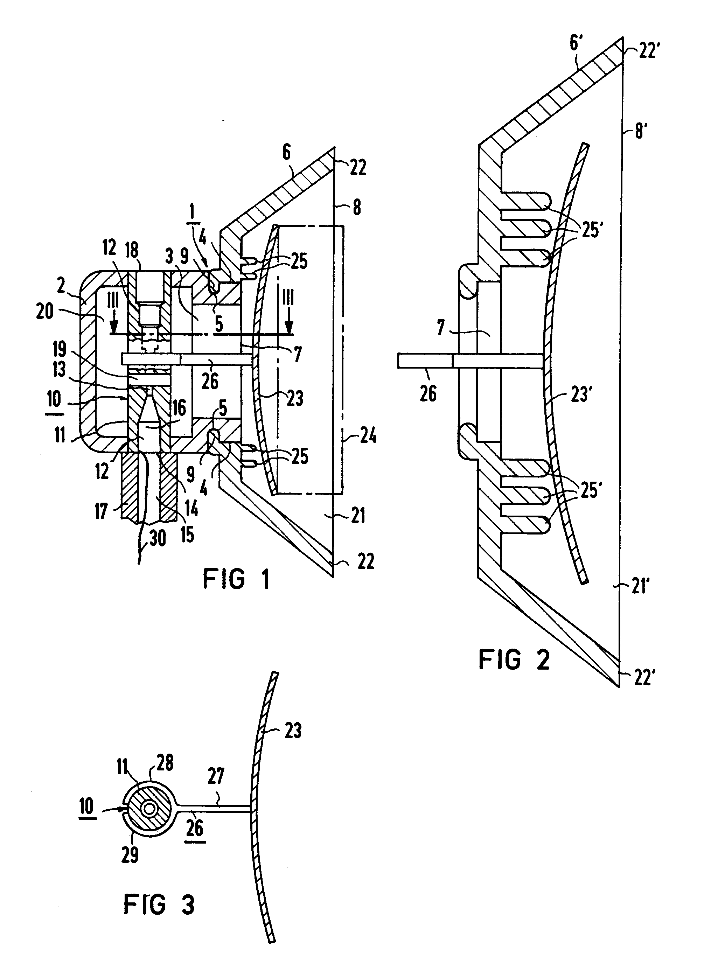

In der Figur 1 umfaßt die Saugelektrode 1 ein napfförmiges Gehäuse 2 (aus Kunststoff) mit einer proximalen Öffnung 3 und einem proximalen Ringwulst 4 mit einer Ringnut 5. Die Saugelektrode 1 umfaßt ferner eine Haube 6 (aus elastischem Kunststoff, z.B. Silikongummi) mit einer distalen Öffnung 7 und einer grösseren proximalen Öffnung 8. Die Haube 6 ist mittels eines zum Ringwulst 4 des Gehäuses 2 passenden distalen Ringwulstes 9 in die Ringnut 5 des Ringwulstes 4 des Gehäuses 2 einknöpfbar. Die Figur 1 zeigt z.B. eine Saugelektrode 1, bei der am napfförmigen Gehäuse 2 eine Haube mit relativ kleiner proximaler Öffnung 8 angeknöpft ist. Anstelle dieser Haube 6 kann auch eine beliebige andere Haube 6' mit z.B. sehr viel größerer proximaler Öffnung 8' angeknöpft werden. Eine solche Haube ist z.B. in der Figur 2 dargestellt.

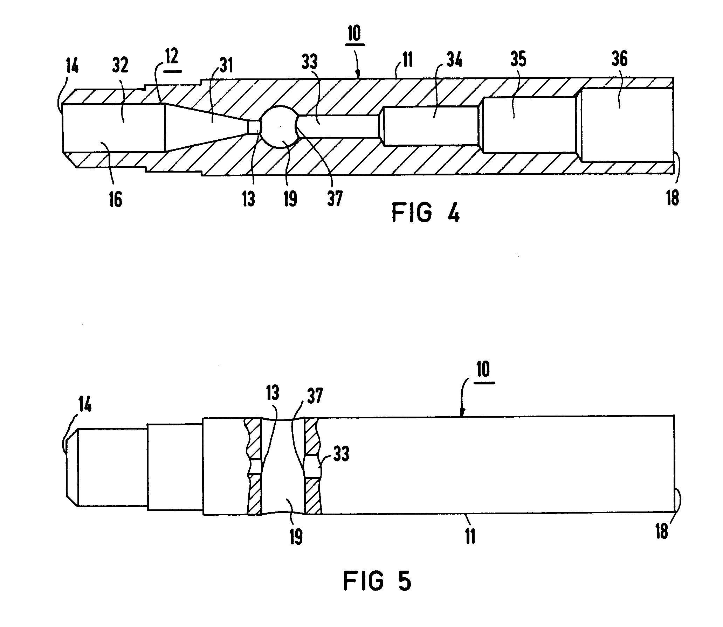

Die Saugelektrode der Figur 1 umfaßt eine Unterdruckdüse 10. Die Unterdrückdüse 10 besteht, wie in den Figuren 4 und 5 noch deutlicher dargestellt ist, aus einem (metallischen) Rohr 11, dessen Rohrkanal 12 an der Stelle 13 enger ist als an den anderen Stellen. Diese enge Stelle 13 dient zur Erzeugung eines Unterdrucks, wenn z.B. vom Rohrende 14 her über einen pneumatischen Anschluß 15 (elektrisch isolierendes) Preßgas durch den mit 16 bezeichneten Eingangsteil des Rohrkanals 12 durch die enge Stelle 13 gepreßt wird. Zum Anschließen einer geeigneten Gaspumpe dient ein pneumatisches Anschlußkabel 17. Der mit 18 bezeichnete Teil des Rohrkanals 12 mündet im Freien. Hier kann also das durch den Rohrkanal 12 gepreßte Gas in die Atmosphäre entweichen.

Gemäß der Figur 1 umfaßt die Unterdruckdüse 10 auch noch einen Ansaugkanal 19, der den Rohrkanal 12 in Höhe der engen Stelle 13 senkrecht durchstößt. Der Ansaugkanal 19 überträgt den an der engen Stelle 13 erzeugten Unterdruck durch den hohlen Innenraum 20 des napfförmigen Gehäuses 2 der Saugelektrode und dessen proximale Öffnung 3 in den hohlen Innenraum 21 der Haube 6. Dadurch saugt sich die Saugelektrode 1 nach Aufsetzen mit dem Rand 22 (bzw. 22') der Haube 6 (bzw. 6') auf die Hautoberfläche (nicht dargestellt) eines zu behandelnden Patienten an der Hautoberfläche fest.

Zum Übertragen eines Reizstromes auf die Hautoberfläche dient eine elektrisch leitende Elektrode 23 (bzw. 23') und ein zwischen Elektrode 23 (bzw. 23') und der Hautoberfläche an der Applikationsstelle einzubringender Schwamm oder Vlies 24, das mit einer geeigneten Elektrodenkontaktflüssigkeit (z.B. Leitungswasser oder Kochsalzlösung) getränkt ist. Die Elektrode 23 (bzw. 23') kann selbstverständlich auch zur Abnahme von physiologischen Signalen, z.B. EKG od.dgl. verwendet werden. Sie ist im vorliegenden Fall plattenförmig ausgebildet und leicht konvex gekrümmt.

Um zu verhindern, daß während des Ansaugvorganges die Elektrode 23 (bzw. 23') den Luftweg von der proximalen Öffnung 3 des napfförmigen Gehäuses 2 bis zur proximalen Öffnung 8 (bzw.

8') der Haube 6 (bzw. 6') verschließt, kann entweder die Elektrode selbst in diesem Bereich mit Öffnungen zur Weiterleitung des Unterdruckes versehen sein oder aber es kann z.B. (wie in der Figur 1 dargestellt) die Haube 6 (bzw. 6') mit Abstandshaltern 25 (bzw. 25') versehen sein, die die Elektrode 23 (bzw. 23') auf Distanz halten.

Die Elektrode 23 (bzw. 23') ist mittels einer elektrisch leitenden (metallischen) Anklemmfeder 26 an der metallischen Unterdruckdüse 10 in der in Figur 1 sowie auch in Figur 3 dargestellten Weise federnd und kontaktierend angeklemmt. Gemäß der Figur 3 umfaßt die Anklemmfeder 26 dazu ein Stielteil 27, das mit der Elektrode 23 verbunden ist und zwei mit dem Stielteil 27 verbundene, sich gabelnde Enden 28, 29, die als Federn ausgebildet sind (bei entsprechender Ausbildung der Anklemmfedern kann aber auch auf das Stielteil verzichtet werden). Diese Art der Befestigung der Elektrode hat den Vorteil, daß zum Reinigen des Ansaugkanals 19 (durch Stechen mit einer feinen Nadel) zuvor die Elektrode 23 (bzw. 23') aus dem Gehäuse 2 mit Haube 6 (6') entfernt werden kann, so daß man freien Zutritt zum Ansaugkanal 19 der Unterdruckdüse 10 hat. Darüber hinaus kann die jeweilige Elektrode jedoch jederzeit leicht durch eine andersartig gestaltete Elektrode, z.B. die Elektrode 23 in Figur 1 durch die in der Figur 2 dargestellte großflächigere Elektrode 23', ausgewechselt werden, wenn nämlich z.B. auch eine größere Haube 6' eingesetzt werden soll. Schließlich ist die beschriebene Art des Anklemmens einer Elektrode 23 bzw. 23' wegen der gelenkigen Verbindung auch noch vorteilhaft, da dadurch die Anpaßfähigkeit der Elektrode an z.B. abnorm gekrümmte Applikationsstellen verbessert wird. Die jeweilige Elektrode 23 bzw. 23' kann von der proximalen Öffnung 8 bzw. 8' der jeweiligen Haube 6 bzw. 6' durch die proximale Öffnung 3 des napfförmigen Gehäuses 2 hindurch an der Unterdruckdüse 10 angeklemmt werden.

Die Anklemmfeder 26 ist im vorliegenden Fall gleichzeitig Kontaktübertrager von der Elektrode 23 (bzw. 23') zum metallischen Rohr 11 der Unterdruckdüse 10. Von dort wird der Kontakt zur Signalleitung 30 weitergeleitet, über die entweder Reizstrom für die Elektrode 23 (bzw. 23') eingespeist wird oder elektrische Signale von der Elektrode abgenommen werden können.

Die Unterdruckdüse 10 selbst kann bei Bedarf im Inneren des napfförmigen Gehäuses 2 auswechselbar eingesetzt sein. Falls eine irreparabel defekte Unterdruckdüse 10 durch eine intakte ersetzt werden soll, so braucht nicht die Unterdruckdüse zusammen mit dem napfförmigen Gehäuse 2 weggeworfen zu werden. Vielmehr braucht lediglich im alten napfförmigen Gehäuse 2 die defekte durch eine intakte Unterdruckdüse ersetzt zu werden.

Beim Verschmutzen des Ansaugkanals 19 (z.B. dadurch, daß angesaugte Elektrodenkontaktflüssigkeit zusammen mit Staub den Ansaugkanal verstopfen) braucht, wie zuvor bereits erwähnt, der Ansaugkanal lediglich mit einer feinen Nadel durchstochen zu werden. Der sich dabei lösende Schmutz wird entweder durchgestoßen oder vor die Mündung der engen Stelle 13 des Rohrkanals 12 gedrückt und von dort vom durchgepumpten Gas durch den Teil 18 des Rohrkanals hindurch in Richtung des frei mündenden Auslaufs mitgerissen.

Die Figuren 4 und 5 zeigen die Unterdruckdüse 10 im vergrößerten Maßstab. Man sieht, daß das Eingangsteil 16 des Rohrkanals 12, das die enge Stelle 13 umfaßt, sich von der engen Stelle 13 in Richtung Rohrende 14 (wo das Preßgas eingedrückt wird) zuerst konusförmig (etwa unter dem Winkel 20°) erweitert (Konuskammer 31) und sich dann mit gleichbleibendem Durchmesser (Zylinderkammer 32) zum Ende 14 hin erstreckt. Der frei mündende Teil 18 des Rohrkanals 12 erweitert sich stufenförmig vom Ansaugstutzen 19 in den vier Zylinderkammern 33, 34, 35 und 36. Der Durchmesser der Zylinderkammer 33, der dem Durchmesser der Mündung 37 am Ansaugstutzen 19 entspricht, ist größer als der Durchmesser der engen Stelle 13 (z.B. etwa doppelt so groß).

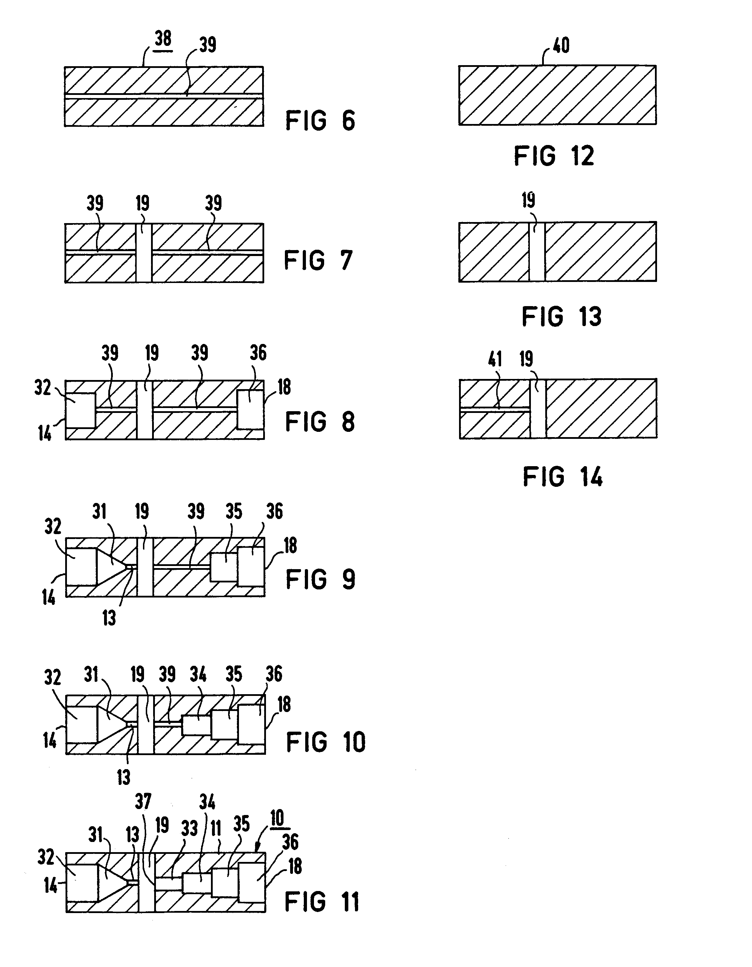

Die Figuren 6 bis 11 zeigen die schrittweise Herstellung einer Unterdruckdüse 10 aus einem Rohrrohling 38, der bereits einen Rohrkanal 39 vom Durchmesser der späteren engen Stelle 13 umfaßt (Figur 6). Gemäß der Figur 7 wird durch diesen Rohrrohling 38 zunächst der Ansaugkanal 19 gebohrt. Dann wird vom Ende 14 her die Kammer 32 und vom Ende 18 her die Kammer 36 gebohrt (Figur 8). Anschließend wird vom Ende 14 her durch die Kammer 32 hindurch die Kammer 31 und vom Ende 18 her durch die Kammer 36 hindurch die Kammer 35 gebohrt. Auf der linken Seite verbleibt jetzt vom ursprünglichen engen Kanal 18 nur noch die enge Stelle 13 (Figur 9). Anschließend werden auf der rechten Seite noch die fehlenden Kammern 34 und 33 nacheinander gebohrt (Figuren 10 und 11).

Die Figuren 12 bis 14 zeigen die Herstellung einer Unterdruckdüse aus einem Rohrrohling 40, der nicht von vornherein einen engen Kanal aufweist (Figur 12). Gemäß Figur 13 wird zuerst wieder der Ansaugkanal 19 gebohrt. Dann wird gemäß Figur 14 auf der linken Seite ein enges Kanalstück 41 gebohrt. Die weitere Verarbeitung erfolgt dann entsprechend den zuvor in den Figuren 8 bis 11 beschriebenen Schritten.

## Patentansprüche

1. Unterdruckdüse für Saugelektroden, bestehend aus einem Rohr (11), dessen Rohrkanal (12) zumindest an einer Stelle (13) enger als an anderen Stellen zur Erzeugung eines Unterdrucks beim Durchführen eines Mediums ausgebildet ist, und mit einem in Höhe der engen Stelle mündenden Ansaugkanal (19), **dadurch gekennzeichnet,** daß der Ansaugkanal (19) das gesamte Rohr (11) an der engen Stelle (13) des Rohrkanals (12) im wesentlichen senkrecht durchstößt, daß sich der Rohrkanal von der engen Stelle (13) in Richtung einer Einführöffnung (14) für das Medium konusförmig erweitert und daß der Durchmesser des Rohrkanals (12) auf der der engen Stelle (13) gegenüberliegenden Seite (37) des Ansaugkanals (19) größer ist als der Durchmesser der engen Stelle (13).

2. Unterdruckdüse nach Anspruch 1, **dadurch gekennzeichnet,** daß sich der Rohrkanal (12) von der engen Stelle (13) in Richtung auf eine Ausführöffnung (18) für das Medium stufenförmig erweitert.

## Claims

1. A low pressure nozzle for suction electrodes, consisting of a tube (11), the tubular channel (12) of which is formed at least at one point (13) to be narrower than at other points for generating a low pressure when conveying a medium, and with an intake channel (19) opening at the height of the narrow point, characterised in that the intake channel (19) penetrates the entire tube (11) at the narrow point (13) of the tubular channel (12) substantially perpendicularly, in that the tubular channel extends in a conical form from the narrow point (13) in the direction of an inlet opening (14) for the medium, and in that the diameter of the tubular channel (12) is greater on the side (37) of the intake channel (19) opposite the narrow point (13) than the diameter of the narrow point (13).

2. A low pressure nozzle according to claim 1, characterised in that the tubular channel (12) extends in stages from the narrow point (13) in the direction of a delivery opening (18) for the medium.

## Revendications

1. Buse à dépression pour des électrodes d'aspiration, constituée par un tube (11), dont le canal (12) est plus étroit au moins en un emplacement (13) qu'en d'autres emplacements pour la création d'une dépression lors du passage d'un fluide, et comportant un canal d'aspiration (19) débouchant au niveau de l'emplacement étroit, caractérisée par le fait que le canal d'aspiration (19) traverse à peu près perpendiculairement l'ensemble du tube (11) au niveau de l'emplacement étroit (13) du canal (12) du tube, que le canal du tube s'élargit en forme de cône à partir de l'emplacement étroit (13) en direction d'une ouverture d'introduction (14) pour le fluide et que le diamètre du canal (12) du tube, sur le côté (37) du canal d'aspiration (19), situé à l'opposé de l'emplacement étroit (13), est supérieur au diamètre de l'emplacement étroit (13).

2. Buse à dépression suivant la revendication 1, caractérisée par le fait que le canal (12) du tube s'élargit avec une forme étagée depuis l'emplacement étroit (13) en direction d'une ouverture de sortie (18) pour le fluide.

FIG 1

FIG 2

FIG 3

FIG 4

FIG 5

FIG 6

FIG 7

FIG 8

FIG 9

FIG 10

FIG 11

FIG 12

FIG 13

FIG 14